(19) Europäisches Patentamt
European Patent Office
Office européen des brevets

(11) Veröffentlichungsnummer: **0 282 455**
**A2**

(12) # EUROPÄISCHE PATENTANMELDUNG

(21) Anmeldenummer: **88810140.9**

(22) Anmeldetag: **07.03.88**

(51) Int. Cl.[4]: **C 07 H 15/04**
A 01 N 43/90, C 12 P 19/44
//(C12P19/44,C12R1:00)

(30) Priorität: **12.03.87 DE 3707955 25.01.88 CH 241/88**

(43) Veröffentlichungstag der Anmeldung:
**14.09.88 Patentblatt 88/37**

(84) Benannte Vertragsstaaten:
**AT BE CH DE ES FR GB GR IT LI LU NL SE**

(71) Anmelder: **Gesellschaft für Biotechnologische Forschung mbH (GBF)**
**Mascheroder Weg 1**
**D-3300 Braunschweig-Stöckheim (DE)**

**CIBA-GEIGY AG**
**Klybeckstrasse 141**
**CH-4002 Basel (CH)**

(72) Erfinder: **Reichenbach, Hans, Prof. Dr.**
**Platanenstrasse 35**
**D-3340 Wolfenbüttel (DE)**

**Höfle, Gerhard, Prof. Dr.**
**Am Windmühlenberg 2**
**D-3300 Braunschweig (DE)**

**Augustiniak, Hermann, Dr.**
**In den Lindendöhren 19**
**D-3340 Wolfenbüttel (DE)**

**Bedorf, Norbert, Dr.**
**Krukenbergstrasse 4**
**D-3307 Königslutter (DE)**

**Forche, Edgar, Dr.**
**Schlosserweg 2**
**D-3300 Braunschweig (DE)**

**Gerth, Klaus, Dr.**
**Am Butterbusch 17**
**D-3300 Braunschweig (DE)**

**Irschik, Herbert, Dr.**
**Masurenweg 15**
**D-3340 Wolfenbüttel (DE)**

**Jansen, Rolf, Dr.**
**Falkenbergstrasse 14**
**D-3300 Braunschweig (DE)**

**Kunze, Brigitte, Dr.**
**Hinter Aegidien 5**
**D-3300 Braunschweig (DE)**

**Sasse, Florenz, Dr.**
**Im Rübenkamp 7**
**D-3300 Braunschweig (DE)**

**Steinmetz, Heinrich**
**Schildweg 44**
**D-3320 Hildesheim-Sorsum (DE)**

**Trowitzsch-Kienast, Wolfram, Dr.**
**Im Dorfe 19**
**D-3300 Braunschweig (DE)**

**Pachlatko, Johannes Paul, Dr.**
**Bölchenstrasse 21**
**D-4411 Seltisberg (DE)**

Der Anmelder hat eine Erklärung nach Regel 28 (4) EPÜ (Herausgabe einer Probe nur an einen Sachverständigen) eingereicht. Eingangsnummer(n) der Hinterlegung(en):NCIB 12411

(54) **Verfahren zur Herstellung einer macrocyclischen Verbindung.**

(57) Macrocyclische Verbindungen der Formel I

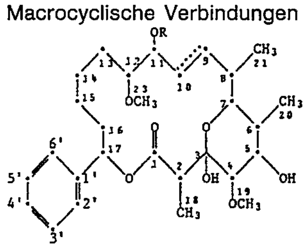

(I)

worin entweder R Methyl bedeutet und in 9,10-Stellung eine Doppelbindung ist, oder worin R Wasserstoff bedeutet und in 9,10-Stellung eine Einfachbindung ist, lassen sich mikrobiologisch durch Kultivierung von Sorangium cellulosum "So ce 26" (NCIB 12 411) gewinnen. Sie stellen Wirkstoffe zur Bekämpfung von Pflanzenkrankheiten dar und lassen sich in üblicher Formulierung anwenden.

EP 0 282 455 A2

**Beschreibung**

Verfahren zur Herstellung einer macrocyclischen Verbindung

Die vorliegende Erfindung betrifft eine macrocyclische Verbindung der Formel I, ein Verfahren zu ihrer Herstellung und ihre Verwendung zur Bekämpfung von Pflanzenkrankheiten sowie pflanzenmikrobizide Mittel, die diese Verbindung als Wirkstoff enthalten.

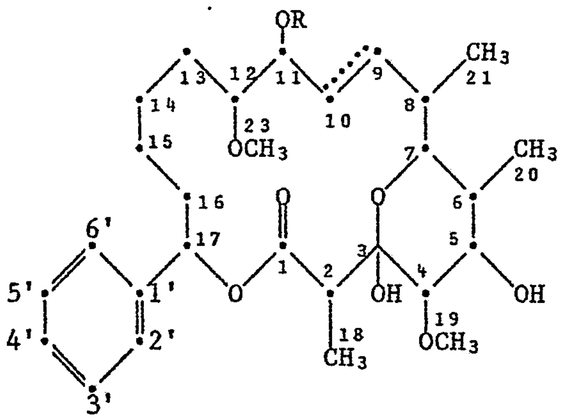

(I)

In dieser Formel bedeutet R Methyl wenn in 9,10-Stellung eine Doppelbindung steht (= Verbindung Ia) oder R bedeutet Wasserstoff, wenn in 9,10-Stellung eine Einfachbindung steht (= Verbindung Ib). Hier und im folgenden soll für die Verbindung Ia auch die Bezeichnung "Soraphen A" und für Ib die Bezeichnung "Soraphen B" verwendet werden. Formel I umfasst grundsätzlich auch die isomeren Strukturen und ihre Gemische untereinander sowie ein beliebiges Gemisch aus Verbindung Ia und Ib. Soraphen A ist bevorzugt.

Verbindungen dieser Körperklasse sind neu. Es gibt in der Literatur keine Hinweise auf eine derartige Struktur.

Die Verbindung der Formel I wird durch mikrobiologische Kultivierung eines Sorangium (Polyangium) cellulosum-Stammes "So ce 26" gewonnen. Dieser Stamm wurde am 5. März 1987 bei der "National Collection of Industrial and Marine Bacteria (NCIB)", Torry Research Station, Aberdeen, Grossbritannien, unter der Nummber NCIB 12 411 gemäss Budapester Vertrag hinterlegt. Sorangium cellulosum gehört zur Ordnung der Myxobacterales, Unterordnung Sorangineae, Familie Polyangiaceae.

Der Mikroorganismus "So ce 26" wurde 1980 aus einer Probe Ziegenmist isoliert, die 1978 auf Djerba, Tunesien gesammelt worden war. "So ce 26" selbst oder Mutanten oder Rekombinanten, die gleichfalls Verbindungen der Formel I produzieren, sind ein weiterer Gegenstand vorliegender Erfindung. Sorangium "So ce 26" lässt sich nach üblichen biologischen Methoden, z.B. in Schüttelkulturen oder in Fermentoren mit Nährmedien kultivieren. Die Fermentationstemperatur beträgt im Regelfall 10-35°C und liegt vorzugsweise bei etwa 30°-32°C, der pH-Wert beträgt 6-8, bevorzugt 7,4. Der Prozess verläuft aerob und unter sterilen Bedingungen.

Die Zusammenmsetzung des Nährmediums kann in grässeren Bereichen variieren. Für essentiell zu assimilierende Nährstoffe müssen eine Kohlenstoff- und eine Stickstoffquelle sowie eine Quelle für anorganische Elemente, die P, S, Mg, K, Ca einschliessen, vorhanden sein.

Fermentierungsprozesse benutzen als C-Quellen bevorzugt Glucose, Stärke und Cellulose, daneben auch Glycerin, Essigsäure und andere. Als N-Quellen sind $NH_4$, $NO_3$ oder auch Peptone geeignet. Eine organische Verbindung als N-Quelle kann nicht gleichzeitig die einzige C-Quelle und Energiequelle bei der Fermentierung sein.

Als Mineralsalze kommen Chloride, Nitrate, Sulfate, Carbonate und Phosphate der Elemente Na, K, $NH_4$, Mg und Ca in Betracht, daneben können Fe, Cu, Mn, Zn, Co und andere als Spurenelemente anwesend sein.

Die Mikroorganismus-Kultur wird in die Schüttelkultur oder in den Fermentor in einer Menge von 0,1-7 % (v/v), bevorzugt 0,5-5 % (v/v), eingesetzt. Die Reaktionsdauer beträgt bei ca. 30°C etwa 2-7 Tage, seltener bis zu 10 Tagen. Für grossvolumige Ansätze werden zweckmässigerweise einleitend kleinere Vorkulturen fermentiert. Die Verwendung von "So ce 26" ist auch in immobilisierter Form möglich, z.B. in Form trägerfixierter Zellen an Alginat.

Es versteht sich, dass der Prozess vorliegender Erfindung nicht auf die Verwendung des "So ce 26" Stammes NCIB 12411 beschränkt ist, sondern jede andere natürliche oder im Labor erzeugte künstliche Mutante oder Rekombinante einschliesst, die in der Lage ist, eine Verbindung der Formel I zu produzieren. Verfahrensmassnahmen zur Herstellung von Mutanten und Rekombinanten eines Mikroorganismus sind dem Fachmann bekannt und geläufig.

Demgemäss betrifft die Erfindung ein Verfahren zur Herstellung der macrocyclischen Verbindungen der Formel I durch Kultivierung des Mikroorganismus Sorangium (Polyangium) cellulosum "So ce 26" (NCIB 12 411) oder einer Mutante oder Rekombinante davon, die ebenfalls die Fähigkeit zur Produktion der Verbindungen der Formel I besitzt, in einem wässrigen Kulturmedium. Im engeren Sinne ist das Verfahren dadurch gekennzeichnet, dass man den Mikroorganismums Sorangium (Polyangium) cellulosum "So ce 26" (NCIB 12 411) in einem Kulturmedium enthaltend mindestens je eine assimilationsfähige C-Quelle und N-Quelle sowie entsprechende anorganische Salze, bei 10-35°C in Anwesenheit oder Abwesenheit eines Adsorberharzes kultiviert, dann die Kulturbrühe bzw. das abfiltrierte Adsorberharz mit einer geeigneten Lösungsmittelphase extrahiert, die erhaltene Lösung einengt und den verbleibenden Rückstand, sofern gewünscht, durch Chromatographie und/oder Umkristallisation reinigt.

Stammkultur und morphologische Beschreibung:

Stammkulturen werden gehalten auf VY/2-Agar (0,5 % Bäckerhefe nach Frischgewicht; 0,1 % $CaCl_2$; 1,5 % Agar; pH 7,2) oder auf Filterpapier über ST21-Agar (0,1 % $KNO_3$; 0,1 % $MgSO_4.7H_2O$; 0,1 % $CaCl_2$; 0,1 % $K_2HPO_4$; 0,01 % $MnSO_4.7H_2O$; 0,02 % $FeCl_3$; 0,002 % Hefe-extrakt; Standard-Spurenelementlösung; 1 % Agar). Die Platten werden bei 30° bebrütet.

Auf Filterpapier über Mineralsalzagar oder auf Hefeagar (VY/2-Agar) bildet der Stamm grosse Schwarmkolonien mit vielen gelborange bis schwarzbraunen Fruchtkörpern. Letztere bestehen aus kleinen Sporangiolen von 15-20 µm Durchmesser, die in mehr oder weniger grossen Haufen dicht gepackt liegen; die Haufen messen meist zwischen 50 und 150 µm im Durchmesser. Die vegetativen Stäbchen haben die für Sorangium typische Form: relativ derbe, im Phasenkontrastmikroskop dunkle, zylindrische Stäbchen mit breit abgerundeten Enden, im Mittel 3-6 µm lang und um 1 µm dick. Nach längerer Adaption an das Wachstum in Flüssigmedien wächst der Stamm in homogener Zellsuspension.

Biologische Charakterisierung des Stammes "So ce 26" (NCIB 12 411)Glukose-Abbau : positiv

Cellulose-Abbau : positiv

Stärke-Abbau : positiv

$NH_4$ als N-Quelle: positiv

$NO_3$ als N-Quelle: positiv

Der Stamm So ce 26 produziert zwei chemisch nahe verwandte Verbindungen Ia and Ib, die das Wachstum zahlreicher Pilze hemmen. Diese macrocyclischen Verbindungen können sowohl aus den Zellen, als auch aus dem Kulturüberstand isoliert werden.

Aus der wässrigen Fermentations-Brühe lässt sich die Verbindung I mit organischen Lösungsmitteln lipophil extrahieren, z.B. mit Ketonen wie Methylethylketon, Cyclohexanon; mit Alkoholen mittlerer Kettenlänge wie Isobutanol, Pentanol, Hexanol; mit Essigsäure-$C_1$-$C_6$-Alkylestern (Ethylacetat, Propylacetat, Butylacetat, Isobutylacetat u.a.); mit Kohlenwasserstoffen oder chlorierten Kohlenwasserstoffen wie Hexan, Toluol, Dichlormethan, 1,2-Dichlorethan, Chlorbenzol, Dichlorbenzol u.a.

Aus dem filtrierten Zellkuchen lässt sich die Verbindung I leicht mit Alkoholen oder Ketonen (z.B. Methanol, Ethanol, Aceton, Methylethylketon) extrahieren.

Aus dem jeweiligen Extrakt kann dann durch Eigengen und/oder Ausfällen die Verbindung der Formel I gewonnen werden, die sich durch fraktionierte Kristallisation oder andere Trennverfahren (z.B. Säulenchromatographie) in die beiden Komponenten Ia und Ib auftrennen lässt.

Die Fermentation zur Herstellung der Verbindung der Formel I wird bevorzugt in Gegenwart eines Adsorberharzes durchgeführt, von dem das gewünschte Produkt aufgenommen wird. Als Adsorberharze kommen vor allem neutrale organische Polymerstoffe in Frage, besonders nicht-ionische hydrophobe Adsorberharze, die zur lipophilen Extraktion geeignet sind. An diesen wird die Verbindung der Formel I fast quantitativ gebunden. Beispiele solcher Harze sind halbpolare Acrylester-Harze, unpolare Polystyrol/Divinylbenzol-Harze und besonders vernetztes Polystyrol. Solche Harze werden in Mengen von 0,1 bis 5 % (v/v) des Fermentations-Volumens zugegeben, bevorzugt 0,5 bis 2 % (v/v). Auch Aktivkohle kommt in Frage.

Eine technisch besonders vorteilhafte Variante zur Gewinnung der Verbindung der Formel I besteht in der Fermentierung mit "So ce 26" (NCIB 12 411) unter den vorstehend angegebenen Bedingungen in Gegenwart von Polystyrol-Harz (z.B. XAD-1180, Rohm and Haas), das vorteilhaft in filtrierbarer Form vorliegt (Körner oder Granulate). Nach Beendigung der Fermentation wird das Harz abfiltriert, mit Wasser gewaschen und mit Methanol oder Ethanol behandelt. Der alkoholische Extrakt wird eingeengt, die durch Zugabe von Diethylether auskristallisierende Verbindung Ia (= Soraphen A) abfiltriert, und das Filtrat wird zur Gewinnung der Verbindung Ib (= Soraphen B) eingeengt und, sofern gewünscht, chromatographisch gereinigt.

Die Erfindung betrifft die Verbindung der Formel I in reiner Form bzw. in kristallisierter Form. Die Erfindung betrifft aber auch Biomassen, Rohextrakte oder Adsorberharze aus der Fermentation, die die Verbindung der Formel I enthalten und als solche oder in weiterformulierter Form zur Bekämpfung von Pflanzenkrankheiten verwendet werden können. Biomassen können auch als gemahlene oder gepresste Trockensubstanzen ("cake") weiterverwendet oder in den Handel gebracht werden.

Die beschriebenen Herstellungsverfahren einschliesslich aller Teilschritte sind ein Bestandteil der vorliegenden Erfindung.

Ueberraschenderweise wurde nun gefunden, dass Verbindungen der Formel I ein für praktische

Bedürfnisse sehr günstiges biozides Spektrum gegen phytopathogene Mikroorganismen, insbesondere gegen Pilze, aufweisen. Sie besitzen sehr vorteilhafte kurative, systemische und insbesondere präventive Eigenschaften under werden zum Schutz von zahlreichen Kultur pflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile vor phytopathogenen Mikroorganismen verschont bleiben.

Als Mikrobizide sind die Wirkstoffe der Formel I z.B. gegen die den folgenden Klassen angehörenden phytopathogenen Pilze wirksam: Fungi imperfecti (z.B. insbesondere Botrytis, ferner Pyricularia, Helminthosporium, Fusarium, Septoria, Cercospora und Alternaria); Basidiomyceten (z.B. Rhizoctonia, Hemileia, Puccinia). Darüber hinaus wirken sie gegen die Klasse der Ascomyceten (z.B. insbesondere Venturia und Erysiphe, ferner Podosphaera, Monilinia, Uncinula) und der Oomyceten (z.B. Phytophthora, Plasmopara). Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Eingeschlossen ist auch ein Verfahren zur Behandlung von Pflanzen, das sich durch Applikation der neuen Verbindungen der Formel I bzw. der diese enthaltenden neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Ananas, Zuckerrohr, Tee, Pfeffer, Weinreben, Hopfen, Bananen- und Naturkautschukgewächse sowie Zierpflanzen (Compositen). Diese Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Dieses Granulat oder ein entsprechendes Pulver kann auch die Trockenmasse der aus dem Fermentor anfallenden Biomasse oder das aus der Fermentationsbrühe abgesiebte und mit den Wirkstoffen der Formel I beladene Adsorberharz sein. Die Verbindungen der Formel I können auch auf Samenkörner aufgebraucht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 2 kg Aktivsubstanz (AS) je ha, bevorzugt bei 50 g bis 500 g AS/ha.

Die Formulierungen d.h. die den Wirkstoff der Formel I gegebenenfalls einen festen oder flüssigen Zusatzstoff enthaltenden Mittel werden in bekannter Weise hergestellt.

Als Lösungsmittel kommen in Frage: Aromatische und aliphatische Kohlenwasserstoffe, wie z.B. Xylolgemische, Cyclohexan oder Paraffine; dann Alkohole und Glykole sowie deren Ether und Ester, wie Ethanol, Ethylenglykol, Ethylenglykolmonomethyl- oder -ethylether, Essigsäureester; Ketone wie Cyclohexanon, stark polare Lösungsmittel, wie N-Methyl-2-pyrrolidon, Dimethylsulfoxid oder Dimethylformamid, sowie

gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermateria lien, z.B. Calcit oder Sand, in Frage. Darüber hinaus kann eine Vielzahl von vorgranulierten Materialien anorganischer Natur wie insbesondere Dolomit oder zerkleinerte Pflanzenrückstände verwendet werden.

Als oberflächenaktive Verbindungen kommen je nach Art des zu formulierenden Wirkstoffs der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Häufiger werden jedoch sog. synthetische Tenside verwendet, insbesondere Alkansulfonate, Fettalkoholsulfate, sulfonierte Benzimidazolderivate oder Alkylsulfonate.

Als nichtionische Tenside kommen Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Als weitere Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Weitere in der Formulierungstechnik gebräuchlichen Tenside sind dem Fachman bekannt oder können der einschlägigen Fachliteratur entnommen werden.

Die agrochemischen Zubereitungen enthalten in der Regel 0,1 bis 95 % Wirkstoff der Formel I, 99,9 bis 5 % eines festen oder flüssigen Zusatzstoffes und 0 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne diesselben einzuschränken.

## 1. Herstellungsbeispiele

### Beispiel H-1: Herstellung der Verbindung der Formel I

Ein 70 Liter Fermentor der Firma Giovanola, Monthey, Schweiz, wird mit 60 Liter Medium (0,1 % Pepton; 0,5 % Glucose; 0,05 % $CaCl_2 \bullet 2H_2O$; 0,05 % $MgSO_4 \bullet 7H_2O$; pH 7,4) gefüllt. Beimpft wird mit 10 Litern einer im gleichen Medium in Schüttelkolben angezogenen Vorkultur (160 Upm, 30° C). Dann wird bei 32° C mit einer Rührgeschwindigkeit von 500 Upm und einer Belüftung von 0,5 $Nm^3$ pro Stunde fermentiert. Die Fermentation dauert etwa 7 Tage. Die Verbindung der Formel I wird während der logarithmischen bis hin zur stationären Wachstumsphase produziert. Sie reichert sich teils in den Zellen, teils im Kulturüberstand an.

Die Fermentationsbrühe wird dann fünfmal mit je 2 Litern Ethylacetat je 5 min. Ausgeschüttelt. Die vereinigten Extrakte werden zweimal mit Wasser gewaschen und im Vakuum eingeengt. Das zurückbleibende dunkle Oel wird über Kieselgel chromatographiert (Lichroprep Si60, Merck, 25-40µm. Laufmittel: Dichlormethan/Aceton in Stufen von 97/3 bis 80/20).

Die Verbindung Ia (= Soraphen A) enthaltenden Fraktionen werden neuerlich eingeengt und ein zweites Mal über Kieselgel chromatographiert (Lichroprep Si60, Merck, 15-25 µm. Laufmittel: Dichlormethan/Aceton 97/3). Das erhaltene Soraphen A kristallisiert aus Diethylether.

Ausbeute: 220 mg.

Die Verbindung Ib (= Soraphen B) enthaltenden Fraktionen werden erneut eingeengt und über den gleichen Kieselgel-Typ (15-25 µm) mit Dichlormethan/Aceton (Gradient: 90/10 bis 85/15) chromatographiert. Die Feinreinigung erfolgt durch eine dritte Chromatographie an einem vernetzten Dextran-Gel wie z.B. Sephadex LH-20 (Pharmacia) mit Methanol.

Ausbeute: 100 mg.

### Beispiel H-2: Herstellung der Verbindung der Formel I in Gegenwart eines Adsorberharzes

Das Verfahren wird in einem Fermentationsvolumen von 300 Litern unter Zugabe von 0,5 % (v/v) Adsorberharz XAD-1180 (Rohm und Haas) durchgeführt. Die Verfahrensbedingungen für diesen vergrösserten Ansatz entsprechen im übrigen denen des Beispiels H-1.

Nach Beendigung der Fermentation wird der Polymerträger abgesiebt, in eine Glassäule gespült, mit 3 Bettvolumen Wasser gewaschen und mit 2 Bettvolumen Methanol eluiert. Das Eluat wird im Vakuum zur Trockne eingeengt und an Kieselgel (Lichroprep Si60 25-40 µm, Merck, Laufmittel: Dichlormethan/Aceton

97/3 in Stufen bis 80/20) chromatographiert.

a) Die das Soraphen A enthaltende Fraktion wird eingeengt und an Kieselgel chromatographiert (Lichroprep Si60, 15-25 µm, Merck. Laufmittel: Dichlormethan/Aceton 97/3.

Wahlweise kann nach der Methanol-Behandlung des Polymerträgers die methanolische Lösung ohne Chromatographie vorsichtig eingeengt werden, wobei das gewünschte Soraphen A nach Zugabe von Diethylether auskristallisiert.

Das Produkt kann aus Diethylether umkristallisiert werden. Ausbeute: 1 g Soraphen A.

b) Die das Soraphen B enthaltende Fraktion wird erneut an Kieselgel chromatographiert (Lichroprep Si60, 15-25 µm, Merck, Laufmittelgradient: Dichlormethan/Aceton 90/10 bis 85/15). Die Feinreinigung erfolgt durch die Chromatographie an Sephadex LH-20 (Pharmacia, Laufmittel: Methanol), Ausbeute: 0,6 g.

<u>Chemische Charakterisierung von Soraphen A (= Verb. Ia)</u>

Summenformel:
$C_{29}H_{44}O_8$. Mg: 520.
Farblose Kristalle aus Diethylether. <u>Fp.</u> 101°C.
$[\alpha]_D^{25}$ = -131° (MeOH); c=1)
<u>UV</u> (MeOH) $\lambda_{max}$ (log ε) = 317 sh (2,03); 267 sh (2,47); 263 (2,59); 257 (2,68); 251 (2,66); 245 sh (2,66); 228 sh (2,84); 215 (3,72); 207 (3,94)
<u>IR</u> (KBr): 975, 992, 1021, 1045, 1071, 1102, 1152, 1187, 1230, 1255, 1272, 1380, 1451, 1708 (sh), 1729, 2854 (sh), 2923 $cm^{-1}$.
<u>MS</u> FAB (neg. Ionen) = 519 $(M-H)^-$
<u>EI</u> (70ev) = 520
Hochauflösung:
ber.: 520,3036
gef.: 520,3020
<u>DC</u> (DC-Alufolie $60F_{254}$, Merck; Laufmittel: Dichlormethan/Aceton 90/10) $R_F$ = 0,5 (Detektion durch Ansprühen mit Anisaldehyd/Schwefelsäure-Reagenz und erhitzen auf 120°C)
<u>HPLC</u> (Säule: 4x250 mm Nucleosil 100-7 $C_{18}$, Macherey Nagel; Fluss 1,5 ml/min; Laufmittel: Methanol/Wasser 70/30 in 20 min linear auf 100/0) $R_t$ - 11,8 min.

<u>Chemische Charakterisierung von Soraphen B (= Verb. Ib)</u>

Summenformel:
$C_{28}H_{44}O_8$. Mg: 508.
$[\alpha]_D^{25}$ = -57° (MeOH; c=1)
<u>UV</u> (MeOH) $\lambda_{max}$ (log ε) = 263 sh (2,98); 256 sh (3,11); 247 sh (3,36); 238 (3,42); 215 sh (3,77); 207 (3,98)
<u>IR</u> (KBr): 713, 973, 994, 1050 (sh), 1071 (sh), 1098, 1154, 1183, 1232 (sh), 1274, 1378, 1459, 1723, 2933, 2952, 3377, 3413, 3451 $cm^{-1}$.
<u>MS</u> FAB (neg. Ionen) = 507 $(M-H)^-$
<u>DC</u> (DC-Alufolie $60F_{254}$, Merck; Laufmittel: Dichlormethan/Aceton 90/10) $R_F$ = 0,3 (Detektion durch Ansprühen mit Anisaldehyd/Schwefelsäure-Reagenz und erhitzen auf 120°C)
<u>HPLC</u> (Säule: 4x250 mm Nucleosil 100-7 $C_{18}$, Macherey Nagel; Fluss 1,5 ml/min; Laufmittel Methanol/Wasser 70/30 in 20 min linear auf 100/0) $R_t$ = 9,0 min.

Die NMR-Daten beider Verbindungen stellen sich wie folgt dar.

Tabelle 1

$^1$H-NMR-Daten ($CDCl_3$; δ in ppm)

| | SORAPHEN A | SORAPHEN B |
|---|---|---|
| 1 | - | - |
| 2 | 3,14 | 3,08 |
| 3 | - | - |
| 4 | 3,18 | 3,12 |
| 5 | 4,04 | 4,04 |
| 6 | 1,94 | 1,82 |
| 7 | 3,83 | 3,92 |
| 8 | 2,51 | 1,88 |
| 9 | 6,19 | 1) |
| 10 | 5,48 | 1,80/1,40 2) |
| 11 | 3,69 | 3,79 3) |
| 12 | 3,43 | 3,35 3) |
| 13A/B | 1,69/1,26 | 1,59/1,35 2) |
| 14A/B | 1,37/1,17 | 1) |
| 15A/B | 1,48 | 1) |
| 16A/B | 1,69/2,10 | 1) |
| 17 | 5,86 | 5,72 |
| 18 | 1,12 | 1,10 |
| 19 | 3,38 | 3,41 |
| 20 | 1,05 | 1,01 |
| 21 | 1,03 | 0,84 |
| 22 | 3,29 | - |
| 23 | 3,44 | 3,36 |
| 1' | - | - |
| 2'/6'<br>3'/5'<br>4' | 7,27-7,38 | 7,25-7,36 |
| 3-OH | 4,43 | 4,68 |
| 5-OH | 3,56 | |

1) : Multipletts 1,25-1,8 ppm

2), 3): Zuordnung jeweils vertauschbar

Tabelle 2

$^{13}C$-NMR-Daten ($CDCl_3$; δ in ppm)

| | SORAPHEN A | SORAPHEN B |
|---|---|---|
| 1 | 170,8 | 171,6 |
| 2 | 46,3 | 45,7 |
| 3 | 99,5 | 99,5 |
| 4 | 76,3 | 76,4 |
| 5 | 68,9 | 69,3[1)] |
| 6 | 35,6 | 35,1 |
| 7 | 42,4 | 71,3 |
| 8 | 35,3 | 32,5 |
| 9 | 139,6 | 27,9[2)] |
| 10 | 122,8 | 27,3[2)] |
| 11 | 85,0 | 68,9[1)] |
| 12 | 82,8 | 69,3[1)] |
| 13 | 30,4 | 25,4[2)] |
| 14 | 23,3 | 24,7[2)] |
| 15 | 25,7 | 22,8[2)] |
| 16 | 35,7 | 34,6 |
| 17 | 74,4 | 75,7 |
| 18 | 11,5 | 11,6 |
| 19 | 57,2 | 57,4[3)] |
| 20 | 10,2 | 10,2 |
| 21 | 12,4 | 14,1 |
| 22 | 56,1 | - |
| 23 | 58,0 | 57,2[3)] |
| 1' | 141,1 | 140,7 |
| 2';6' | 126,2 | 126,4 |
| 3';5' | 128,5 | 128,4 |
| 4' | 128,0 | 128,0 |

1); 2); 3): Zuordnung jeweils vertauschbar

Aufgrund der Röntgenstrukturanalyse und der vorstehend ermittelten Daten werden für Soraphen A (= Verb. Ia) und für Soraphen B (= Verb. Ib) die folgenden räumlichen Strukturen Ia′ und Ib′ angenommen:

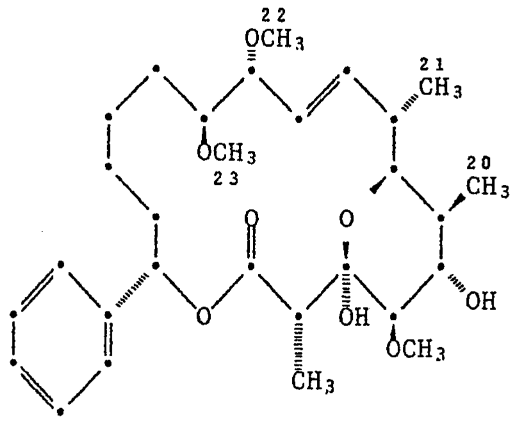

(Ia')

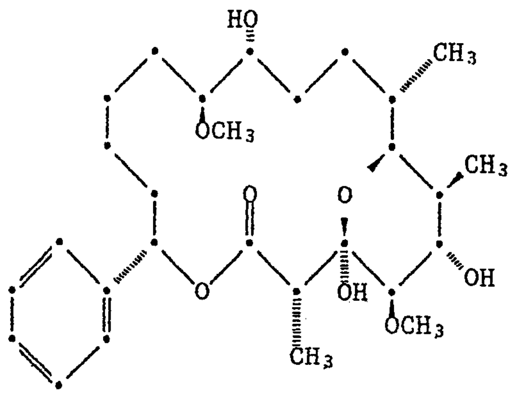

(Ib')

Die Erfindung schliesst insbesondere auch die Verbindungen Ia′ und Ib′, ihre Herstellung und ihre Verwendung als Pflanzenmikrobizide ein.

Wirkungsspektrum der Verbindungen Ia und Ib im Plattendiffusionstest

Die Konzentration der Verbindungen beträgt 1 µg/Testblättchen, das Agarvolumen 10 ml pro Platte (Petrischalen von 9 cm Durchmesser). Für Ia und Ib wurden die gleichen Werte ermittelt.

| Testorganismus | Hemmhofdurchmesser [mm] |
|---|---|
| Debaryomyces hansenii | 29 |
| Candida albicans | 25 |
| Hansenula anomala | 15 |
| Nadsonia fulvescens | -- |
| Nematospora coryli | 35 |

| | |
|---|---|
| Rhodotorula glutinis | 21 |
| Saccaromyces cerevisiae | 20 |
| Schizosaccharomyces pombe | -- |
| Torulopsis glabrata | -- |
| Alternaria solani | 27 |
| Botrytis cinerea | 30 |
| Mucor hiemalis | 30 |
| Pythium debaryanum | 45 |
| Rhizopus arrhizus | 10 |

Auch die minimale Hemmkonzentration MIC ergibt bei beiden Verbindungen Ia und Ib die gleichen Werte.

Minimale Hemmkonzentrationen

| Organismus | MNK/µg/ml |
|---|---|
| Nematospora coryli | 0,05 |
| Candida albicans | 0,06 |
| Rhodotorula glutinis | 1,0 |
| Saccharomyces cerevisiae | 2,0 |
| Torulopsis glabrata | 3,0 |
| Nadsonia fulvescens | 3,0 |
| Mucor hiemalis | 0,03 |

Die Hemmwirkung gegenüber Candida ist auch nach 24-stündiger Einwirkungsdauer noch reversibel. Die Vermehrung von Nematospora coryli kommt 90 Minuten nach Zugabe der Verbindung I zum Erliegen. Zur gleichen Zeit werden die DNS- und die Proteinsynthese eingestellt.

Serumbindung: Rinderserum hat keinen Einfluss auf die Wirksamkeit der Verbindung I.

2. Formulierungsbeispiele für den Wirkstoff der Formel I (% = Gewichtsprozent)

| 2.1 Emulsions-Konzentrate | a) | b) | c) |
|---|---|---|---|
| Wirkstoff | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenol-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2 Lösungen | a) | b) | c) | d) |
|---|---|---|---|---|
| Wirkstoff | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |
| (MG = Molekulargewicht) | | | | |

Die Lösungen sind zur Anwendung in Form kleinster Tropfen geeignet.

| 2.3 Granulate | a) | b) |
|---|---|---|
| Wirkstoff | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht un das Lösungsmittel anschliessend im Vakuum abgedampft.

| 2.4 Stäubemittel | a) | b) |
|---|---|---|
| Wirkstoff | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Diese lassen sich durch weiteren Zusatz der drei Trägerstoffe auf anwendungsfertige Stäube mit 0,001 % Wirkstoff vermahlen.

| 2.5 Spritzpulver | a) | b) | c) |
|---|---|---|---|
| Wirkstoff | 25 % | 50 % | 75 % |
| Na-Ligninsulfonat | 5 % | 5 % | - % |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - % |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2.6 Umhüllungs-GranulatWirkstoff 3 %
Polyethylenglykol (MG 200) 3 %
Kaolin 94 %
(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.7 Suspensions-KonzentratWirkstoff 40 %
Ethylenglykol 10 %
Nonylphenolpolyethylenglykolether (15 Mol Ethylenoxid) 6 %
N-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

2.8 Biomasse-Konzentrat

Die aus der Produktionskultur anfallende Biomasse wird getrocknet, vermahlen und im Gewichtsverhältnis 80:20 mit Ethylenglykol-monomethylether gemischt. Ein solches Konzentrat lässt sich beliebig mit Wasser zu Sprühsuspensionen verdünnen.

3. Biologische Beispiele an Pflanzen

(Im folgenden bedeutet "Wirkstoff" ohne weiteren Hinweis sowohl "Soraphen A" wie "Soraphen B")

Beispiel 3.1: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

b) Systemische Wirkung

Zu Weizenpflanzen wird 5 Tage nach der Aussaat eine aus Spritzpulver des Wirkstoffes hergestellte Spritzbrühe gegossen (0,002 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

Der Pilzbefall wurde in beiden Versuchen vollständig durch den Wirkstoff gehemmt.

Unbehandelte aber infizierte Kontrollpflanzen wiesen dagegen einen Puccinia-Befall von 100 % auf.

Beispiel 3.2: Wirkung gegen Phytophthora auf Tomatenpflanzen

a) Residual-protektive Wirkung

Tomatenpflanzen wurden nach 3-wöchiger Anzucht mit einer aus Spritzpulver des Wirkstoffes hergestellten Spritzbrühe (0,02 % Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luchtfeuchtigkeit und 20°C.

b) Systemische Wirkung

Zu Tomatenpflanzen wurde nach 3-wöchiger Anzucht eine aus Spritzpulver des Wirkstoffes hergestellte Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20°C.

In beiden Versuchen wurde bei der Auswertung kein Pilzbefall beobachtet.

Beispiel 3.3: Wirkung gegen Plasmopara viticola auf Reben

Residual-protektive Wirkung

Im 4-5 Blattstadium werden Rebensämlinge mit einer aus Spritzpulver des Wirkstoffes hergestellten Spritzbrühe (0,006 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20°C wird der Pilzbefall beurteilt.

Im Gegensatz zu den unbehandelten, aber infizierten Kontrollpflanzen mit 100 % Pilzbefall waren die mit dem Wirkstoff I behandelten Pflanzen befallsfrei.

Beispiel 3.4: Wirkung gegen Cercospora arachidicola auf Erdnusspflanzen

Residual protektive Wirkung

10-15 cm hohe Erdnusspflanzen werden mit einer aus Spritzpulver der Wirksubstanz hergestellten Spritzbrühe (0,006 % Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Die mit dem Wirkstoff I behandelten Pflanzen waren ohne Befall. Die mit Soraphen A behandelten Pflanzen zeigten auch bei Anwendung einer Spritzkonzentration von 0,002 % am Schluss der Auswertung keinen Befall. Unbehandelte aber infizierte Kontrollpflanzen wiesen dagegen einen Cercospora-Befall von 100 % auf.

Beispiel 3.5: Wirkung gegen Venturia inaequalis auf Apfeltrieben

Residual-protektive Wirkung

Apfelstecklinge mit 10-20 cm langen Frischtrieben werden mit einer aus Spritzpulver des Wirkstoffes hergestellten Spritzbrühe (0,02 %) Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt.

Die mit dem Wirkstoff behandelten Stecklinge waren befallsfrei.

Beispiel 3.6: Wirkung gegen Botrytis cinerea an Apfelfrüchten

Residual-protektive Wirkung

Künstlich verletzte Aepfel werden behandelt, indem eine aus Spritzpulver der Wirksubstanz hergestellte Spritzbrühe (0,02 % Aktivsubstanz) auf die Verletzungsstellen aufgetropft wird. Die behandelten Früchte

werden anschliessend mit einer Sporensuspension des Pilzes inokuliert und während einer Woche bei hoher Luftfeuchtigkeit und ca. 20°C inkubiert. Bei der Auswertung werden die angefaulten Verletzungsstellen gezählt und daraus die fungizide Wirkung der Testsubstanz abgeleitet.

Der Wirkstoff hemmte den Pilzwuchs vollständig.

Beispiel 3.7: Wirkung gegen Erysiphae graminis auf Gerste

a) Residual-protektive Wirkung

Ca. 8 cm hohe Gerstenpflanzen werden mit einer aus Spritzpulver des Wirkstoffes hergestellten Spritzbrühe (0,006 % Aktivsubstanz) besprüht. Nach 3-4 Studen werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,002 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Die Pflanzen waren in beiden Versuchen befallsfrei, die Kontrollpflanzen vollständig befallen.

Beispiel 3.8: Wirkung gegen Rhizoctonia solani (Bodenpilz auf Reispflanzen)

Protektiv-lokale Bodenapplikation

12 Tage alte Reispflanzen werden mit einer aus einer Formulierung des Wirkstoffes hergestellten Spritzbrühe (0,002 % Aktivsubstanz), ohne oberirdische Pflanzenteile zu kontaminieren, angegossen. Zur Infizierung der behandelten Pflanzen wird eine Suspension von Myzel und Sklerotien von R. solani auf die Bodenoberfläche gegeben. Nach 6-tägiger Inkubation bei 27°C (Tag), bzw. 23°C (Nacht) und 100 % rel. Luftfeuchtigkeit (Feuchtkasten) in der Klimakammer wird der Pilzbefall auf Blattscheide, Blättern und Stengel beurteilt.

Nach Behandlung mit dem Wirkstoff trat kein Befall auf.

**Patentansprüche**

1. Macrocyclische Verbindung der Formel I

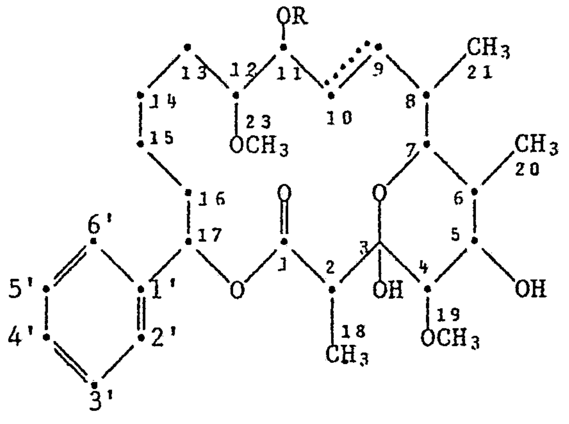

(I)

worin entweder R Methyl bedeutet und in 9,10-Stellung eine Doppelbindung ist, oder worin R Wasserstoff bedeutet und in 9,10-Stellung eine Einfachbindung ist.

2. Die macrocyclischen Verbindungen der Formeln Ia′ und Ib′

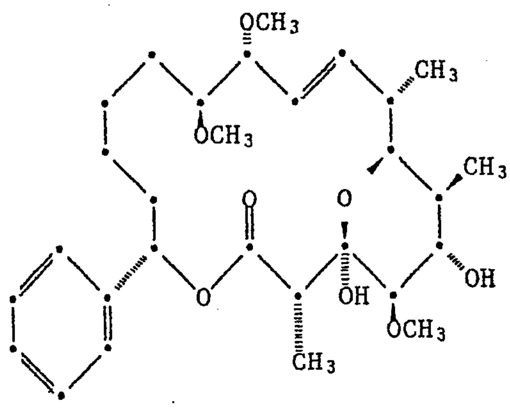

(Ia')

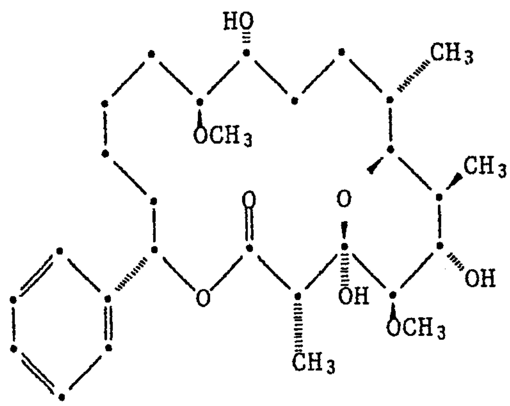

(Ib')

3. Eine macrocyclische Verbindung Ia mit der Summenformel $C_{29}H_{44}O_8$, einem Drehwert $[\alpha]_D^{25} = -131°$ (MeOH; c = 1) und den folgenden NMR-Spektren:

| $^1H$-NMR-Daten ($CDCl_3$; δ in ppm) | | $^{13}C$-NMR-Daten ($CDCl_3$; δ in ppm) | |
|---|---|---|---|
| 3,14 | 1,69/2,10 | 170,8 | 25,7 |
| 3,18 | 5,86 | 46,3 | 35,7 |
| 4,04 | 1,12 | 99,5 | 74,4 |
| 1,94 | 3,38 | 76,3 | 11,5 |
| 3,83 | 1,05 | 68,9 | 57,2 |
| 2,51 | 1,03 | 35,6 | 10,2 |
| 6,19 | 3,29 | 42,4 | 12,4 |
| 5,48 | 3,44 | 35,3 | 56,1 |
| 3,69 | 7,27-7,38 | 139,6 | 58,0 |
| 3,43 | 4,43 | 122,8 | 141,1 |
| 1,69/1,26 | 3,56 | 85,0 | 126,2 |
| 1,37/1,17 | | 82,8 | 128,5 |
| 1,48 | | 30,4 | 128,0 |
| | | 23,3 | |

4. Eine macrocyclische Verbindung Ib mit der Summenformel $C_{28}H_{44}O_8$, einem Drehwert $[\alpha]_D^{25}$ = -57° (MeOH; c = 1) und den folgenden NMR-Spektren:

| $^1H$-NMR-Daten ($CDCl_3$; δ in ppm) | | $^{13}C$-NMR-Daten ($CDCl_3$; δ in ppm) | |
|---|---|---|---|
| | 1) | | |
| 3,08 | 1) | 171,6 | 24,7 2) |
| 3,12 | 1) | 45,7 | 22,8 2) |
| 4,04 | 5,72 | 99,5 | 34,6 |
| 3,92 | 1,10 | 76,4 | 75,7 |
| 1,88 | 3,41 | 69,3 1) | 11,6 |
| 1) | 1,01 | 35,1 | 57,4 3) |
| 1,80/1,40 2) | 0,84 | 71,3 | 10,2 |
| 3,79 3) | 3,36 | 32,5 | 14,1 |
| 3,35 3) | 7,25-7,36 | 27,9 2) | 57,2 3) |
| 1,59/1,35 2) | 4,68 | 27,3 2) | 140,7 |
| | | 68,9 1) | 126,4 |
| | | 69,3 1) | 128,4 |
| | | 25,4 2) | 128,0 |

1) : Multipletts 1,25-1,8 ppm
2); 3): Zuordnung jeweils vertauschbar

1); 2); 3): Zuordnung jeweils vertauschbar

5. Verfahren zur Herstellung der macrocyclischen Verbindungen gemäss einem der Ansprüche 1 bis 4, gekennzeichnet durch Kultivierung des Mikroorganismus Sorangium (Polyangium) cellulosum "So ce 26" (NCIB 12 411) oder einer Mutante oder Rekombinante davon, die ebenfalls die Fähigkeit zur Produktion der Verbindungen der Formel I besitzt, in einem wässrigen Kulturmedium.

6. Verfahren gemäss Anspruch 5, dadurch gekennzeichnet, dass man den Mikroorganismums Sorangium (Polyangium) cellulosum "So ce 26" (NCIB 12 411) in einem Kulturmedium enthaltend mindestens je eine assimilationsfähige C-Quelle und N-Quelle sowie entsprechende anorganische Salze, bei 10-35°C in Anwesenheit oder Abwesenheit eines Adsorberharzes kultiviert, dann die Kulturbrühe bzw. das abfiltrierte Adsorberharz mit einer geeigneten Lösungsmittelphase extrahiert, die erhaltene Lösung einengt und den verbleibenden Rückstand, sofern gewünscht, durch Chromatographie und/oder Umkristallisation reinigt.

7. Verfahren gemäss Anspruch 6, dadurch gekennzeichnet, dass man
- Sorangium (Polyangium) cellulosum "So ce 26" (NCIB 12 411) in einem C-Quellen, N-Quellen und Mineralsalze enthaltenden Kulturmedium in Gegenwart eines nicht-ionischen hydrophoben Adsorberharzes kultiviert,
- das vom Kulturmedium abgetrennte Adsorberharz mit einer alkoholischen Phase eluiert,
- das Eluat zur Trockne einengt und gegebenenfalls ferner
- in einem polaren Lösungsmittel oder Lösungsmittel-Gemisch aufnimmt,
- an Kieselgel chromatographiert, und sofern gewünscht,
- die Fraktion mit der Verbindung Ia zur Trockne einengt und reines Ia durch Imkristallisieren aus Diethylether gewinnt und/oder, sofern gewünscht,
- die Fraktion mit der Verbindung Ib zur Trockne einengt, in einem alkoholischen Medium aufnimmt, an einem Absorberharz auf Basis von vernetztem Dextran-Gel chromatographiert und gegebenenfalls die Fraktion mit der Verbindung Ib zur Trockne einengt.

8. Verfahren gemäss Anspruch 6, dadurch gekennzeichnet, dass man
- Sorangium (Polyangium) cellulosum "So ce 26" (NCIB 12 411) in einem C-Quellen, N-Quellen und Mineralsalze enthaltenden Kulturmedium kultiviert,
- das Kulturmedium mit einem Essigsäureester und gegebenenfalls die Zellen mit einem Alkohol oder

Keton eluiert,
- das Eluat einengt, und den Rückstand gegebenenfalls in einem polaren Lösungsmittel oder Lösungsmittel-Gemisch aufnimmt, an Kieselgel chromatographiert, und
- die Fraktion mit der Verbindung IA einengt und Verbindung IA durch Umkristallisieren aus Diethylether reinigt, und/oder gegebenenfalls
- die Fraktion mit der Verbindung Ib einengt, in einem alkoholischen Medium aufnimmt und an einem Adsorberharz auf Basis von vernetztem Dextran-Gel chromatographiert.

9. Mittel zur Bekämpfung von Pflanzenkrankheiten enthaltend als Wirkstoff mindestens eine Verbindung der Formel I gemäss Anspruch 1, zusammen mit einem geeigneten Trägermaterial.

10. Mittel gemäss Anspruch 9 enthaltend als Wirkstoff die Verbindung Ia′ und/oder Ib′ gemäss Anspruch 2.

11. Mittel gemäss Anspruch 9, dadurch gekennzeichnet, dass der wesentliche Bestandteil die nach dem Verfahren des Anspruchs 5 gewonnnene Biomasse mit der Verbindung der Formel I ist.

12. Verwendung der macrocyclischen Verbindungen gemäss einem der Ansprüche 1-4 zur Bekämpfung von Pflanzenkrankheiten.

13. Eine biologisch reine Kultur des Mikroorganismus Sorangium (Polyangium) cellulosum "So ce 26" (NCIB 12 411) oder eine Verbindung I produzierende Mutante oder Rekombinante davon.

14. Sorangium cellulosum "So ce 26" (NCIB 12 411).